# EUROPEAN PATENT APPLICATION

(11) **EP 4 360 688 A1**
(43) Date of publication of application: **01.05.2024**
(21) Application number: 22828354.5
(22) Date of filing: 17.06.2022
(51) Int. Cl.: A61M 25/09

(54) **GUIDE WIRE**

(30) Priority: 22.06.2021 JP 2021103270
(71) Applicant: Asahi Intecc Co., Ltd., Seto-shi, Aichi 489-0071 (JP)
(72) Inventor: TSUZUKU, Seiji, Seto-shi, Aichi 489-0071 (JP); KOSEKI, Kuon, Seto-shi, Aichi 489-0071 (JP); USHIDA, Keisuke, Seto-shi, Aichi 489-0071 (JP)
(74) Representative: Prinz & Partner mbB
(86) International application number: PCT/JP2022/024369
(87) International publication number: WO 2022/270437

(57) **Abstract**

A guide wire that can be prevented from being caught by a convex portion provided on an inner peripheral surface of an inserted member or a convex portion of a blood vessel inner wall or the like is provided.

A guide wire 1 includes a core shaft 2 having a distal end portion curved in a predetermined direction, a coil body 3 covering the outer periphery of the core shaft 2, and a distal tip 10 joining a distal end portion 2A of the core shaft 2 and a distal end portion of the coil body 3. The distal tip 10 has a convex shape toward a distal end side, and includes, in the longitudinal section of the core shaft 2, a curve inner portion 11 located on an inside of the curve of the core shaft 2 relative to the distal end of the core shaft 2 and a curve outer portion 12 located on an outside of the curve of the core shaft 2 relative to the distal end of the core shaft 2. A cross-sectional area of the curve inner portion 11 is smaller than that of the curve outer portion 12.

## Description

### TECHNICAL FIELD

The present inventions relates to a guide wire.

### BACKGROUND ART

When treating stenosis that occurs in the blood vessels such as the coronary arteries surrounding the heart, or when treating a region where the blood vessel is completely occluded (e.g., chronic total occlusion: CTO, etc.) due to the progression of calcification, a guide wire is inserted into the blood vessel to guide therapeutic instruments such as a balloon catheter.

As the above-mentioned guide wire, for example, there is proposed a guide wire in which a cutting edge, whose diameter is gradually reduced toward the distal end side, is formed on the outer peripheral portion of the distal tip to scrape off an occluded part (e.g., see Patent literature 1).

### CITATION LIST

### Patent Literature

Patent Literature 1: Japanese Patent No. 4308801

### SUMMARY OF INVENTION

### TECHNICAL PROBLEM

However, when the guide wire in Patent literature 1, with its distal end portion at an angle, is inserted into a catheter, the guide wire may be caught by a convex portion of a marker or the like provided on the inner peripheral surface of the catheter or a convex portion of the blood vessel inner wall or the like and may not be able to pass through the convex portion.

The present invention has been made based on the above circumstances, and the object of the present invention is to provide a guide wire that can be prevented from being caught by a convex portion provided on an inner peripheral surface of a member to be inserted or a convex portion of a blood vessel inner wall or the like.

### SOLUTION TO PROBLEM

In order to achieve the above object, a guide wire according to one aspect of the present invention includes a core shaft having a distal end portion which is curved in a predetermined direction, a tubular body covering the outer periphery of the core shaft, and a distal tip joining the distal end portion of the core shaft and a distal end portion of the tubular body. The distal tip has a convex shape toward a distal end side, and includes, in the longitudinal section of the core shaft, a curve inner portion located on an inside of the curve of the core shaft relative to the distal end of the core shaft and a curve outer portion located on an outside of the curve of the core shaft relative to the distal end of the core shaft. A cross-sectional area of the curve inner portion is smaller than a cross-sectional area of the curve outer portion.

An outer shape of the curve inner portion on a distal end side may include a first arcuate portion having a first curvature, and an outer shape of the curved outer portion on a distal end side may include a second arcuate portion having a second curvature, and the first curvature is smaller than the second curvature.

Provided that the length from the distal end of the tubular body to the distal end surface of the distal tip is defined as the projection length, the distal tip may include a distal portion provided in the curve outer portion and having the longest projection length, and a proximal portion provided in the curve inner portion and having a shorter projection length than that of the distal portion, and the projection length becomes shorter from the distal portion toward the proximal portion.

The distal tip may include a flat portion, and the flat portion may be inclined relative to an axis of the distal end portion of the core shaft so that a part of the flat portion on a side of the curve inner portion is closer to the tubular body than a part of the flat portion on a side of the curve outer portion.

### EFFECT OF THE INVENTION

According to the present invention, it is possible to provide a guide wire that can be prevented from being caught by a convex portion provided on an inner peripheral surface of an inserted member or a convex portion of a blood vessel inner wall or the like.

### BRIEF DESCRIPTION OF DRAWINGS

FIG. 1 is a partial cross-sectional view of a guide wire according to a first embodiment.
FIG. 2 is a front view of a distal tip in FIG. 1.
FIG. 3 is a diagram illustrating a state in which the guide wire of FIG. 1 is inserted into a catheter.
FIG. 4 is a partial cross-sectional view of a guide wire according to a second embodiment.
FIG. 5 is an explanatory diagram of how the guide wire of FIG. 4 is used.
FIG. 6 is an explanatory diagram of how the guide wire of FIG. 4 is used.
FIG. 7 is an explanatory diagram of a guide wire of a modification example.

### EMBODIMENTS OF THE INVENTION

A guide wire according to an embodiment of the present invention will be described below with reference to the drawings. However, the present invention is not limited only to the embodiments described in the drawings. Note that, in the present disclosure, a distal end means an end portion of the guide wire where a distal tip is located, and a proximal end means the other end portion on a side opposite to the distal end.

### <First embodiment>

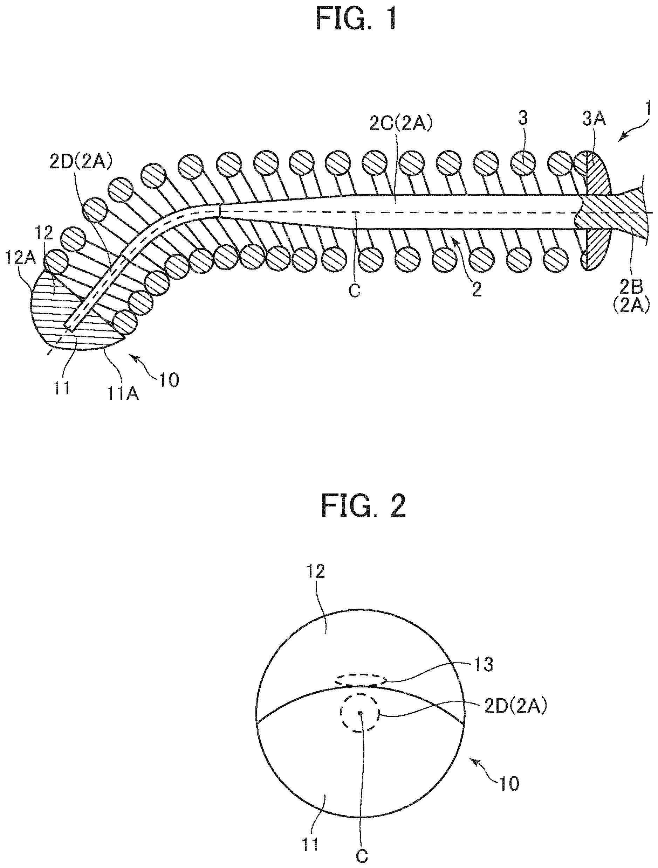

FIG. 1 is a partial cross-sectional view of a guide wire 1 according to a first embodiment. As shown in FIG. 1, the guide wire 1 includes a core shaft 2, a coil body 3, and a distal tip 10.

The core shaft 2 is a shaft extending from the proximal end to the distal end of the guide wire 1. The core shaft 2 includes a distal end portion 2A located on a distal end side and a proximal end portion, not shown, located on a proximal end side of the distal end portion 2A. The distal end portion 2A is configured to gradually decrease in diameter from the proximal end toward the distal end. The distal end portion 2A includes a tapered portion 2B, an intermediate portion 2C, and a most distal end portion 2D.

The proximal end portion, not shown, is connected to the proximal end of the tapered portion 2B, and the intermediate portion 2C is connected to the distal end of the tapered portion 2B. A distal end portion of the intermediate portion 2C has a reduced diameter. The most distal end portion 2D is connected to the distal end of the intermediate portion 2C. The most distal end portion 2D has a substantially cylindrical shape, and its distal end is connected to the distal tip 10. The most distal end portion 2D is curved in a predetermined direction. That is, the most distal end portion 2D of the distal end portion 2A is at an angle. This makes it easier for the guide wire 1 to select a branch vessel in the blood vessel or the like.

Examples of the material for forming the core shaft 2 include a metal material such as stainless steel such as SUS304, a Ni-Ti alloy, or a Co-Cr alloy. The total length of the core shaft 2 is, for example, 1,800 to 3,000 mm, the length of the distal end portion 2A is, for example, 80 to 550 mm, and the length of the most distal end portion 2D is, for example, 0 to 40 mm. The outer diameter of the proximal end portion, not shown, is, for example, 0.2 to 1.0 mm, and the outer diameter of the most distal end portion 2D is, for example, 0.03 to 0.15 mm.

The coil body 3 is provided around the intermediate portion 2C and the most distal end portion 2D of the core shaft 2. The coil body 3 is formed in a hollow cylindrical shape by spirally winding a metal wire around the core shaft 2. The distal end of the coil body 3 is joined to the distal tip 10, and the proximal end of the coil body 3 is joined to the intermediate portion 2C by a joint portion 3A. The joint portion 3A is formed by, for example, a brazing material (aluminum alloy brazing, silver brazing, gold brazing, etc.), metal solder (an Ag-Sn alloy, an Au-Sn alloy, etc.), an adhesive (an epoxy-based adhesive, etc.), or the like.

The metal wire for forming the coil body 3 is one or more single wires or one or more twisted wires. A diameter of the metal wire is, for example, 0.01 to 0.10 mm. Examples of the material for forming the metal wire of the coil body 3 include stainless steel such as SUS316, a superelastic alloy such as a Ni-Ti alloy, and radiopaque metal such as platinum or tungsten.

The distal tip 10 has a substantially hemispherical shape, is provided at the distal end of the guide wire 1, and joins the distal end of the core shaft 2 and the distal end of the coil body 3. The distal tip 10 is formed by, for example, a brazing material (aluminum alloy brazing, silver brazing, gold brazing, etc.), metal solder (an Ag-Sn alloy, an Au-Sn alloy, etc.), an adhesive (an epoxy-based adhesive, etc.), or the like.

The distal tip 10 has a convex shape toward the distal end side. The distal tip 10 has a convex shape toward the distal end side. In the longitudinal section of the core shaft 2 (cross section cut along the plane including a central axis C of the core shaft 2), the distal tip 10 includes a curve inner portion 11 located on the inside of the curve of the core shaft 2 relative to the distal end (the center of the distal end) of the core shaft 2 and a curve outer portion 12 located on the outside of the curve of the core shaft 2 relative to the distal end (the center of the distal end) of the core shaft 2. In the distal tip 10 shown in FIG. 1, a part located on the inside of the curve of the core shaft 2 relative to the central axis C corresponds to the curve inner portion 11, and a part located on the outside of the curve of the core shaft 2 corresponds to the curve outer portion 12. The cross-sectional area of the curve inner portion 11 is configured to be smaller than the cross-sectional area of the curve outer portion 12.

In the present embodiment, the outer shape of the curve inner portion 11 on the distal end side includes a first arcuate portion 11A having a first curvature R1. The outer shape of the curve outer portion 12 on the distal end side includes a second arcuate portion 12A having a second curvature R2. The first curvature R1 is configured to be smaller than the second curvature R2.

The curve inner portion 11 is formed in the distal tip 10 by, for example, forming a hemispherical distal tip corresponding portion and then shaving a part of the distal tip corresponding portion from the most distal end portion to a part of the proximal end, and its surroundings. The most distal end portion 2D of the core shaft 2 is bent to the side where the distal tip 10 is shaved, so that the most distal end portion 2D of the core shaft 2 can be at an angle.

FIG. 2 is a front view of the distal tip 10. As shown in FIG. 2, the distal tip 10 includes the curve inner portion 11. Thus, a part of the distal tip 10 on the extension of the most distal end portion 2D is not the most distal end of the distal tip 10, but a region 13 portion of the distal tip 10 is the most distal end of the distal tip 10.

As described above, the guide wire 1 in the first embodiment includes the core shaft 2 in which the most distal end portion 2D of the distal end portion 2A is curved in a predetermined direction, the coil body 3 that covers the outer periphery of the core shaft 2, and the distal tip 10 that joins the distal end portion 2A of the core shaft 2 and the distal end portion of the coil body 3. The distal tip 10 has a convex shape toward the distal end side, and includes the curve inner portion 11 located on the inside of the curve of the core shaft 2 relative to the distal end of the core shaft 2 and the curve outer portion 12 located on the outside of the curve of the core shaft 2 relative to the distal end of the core shaft 2, in the longitudinal section of the core shaft 2. The cross-sectional area of the curve inner portion 11 is configured to be smaller than the cross-sectional area of the curve outer portion 12.

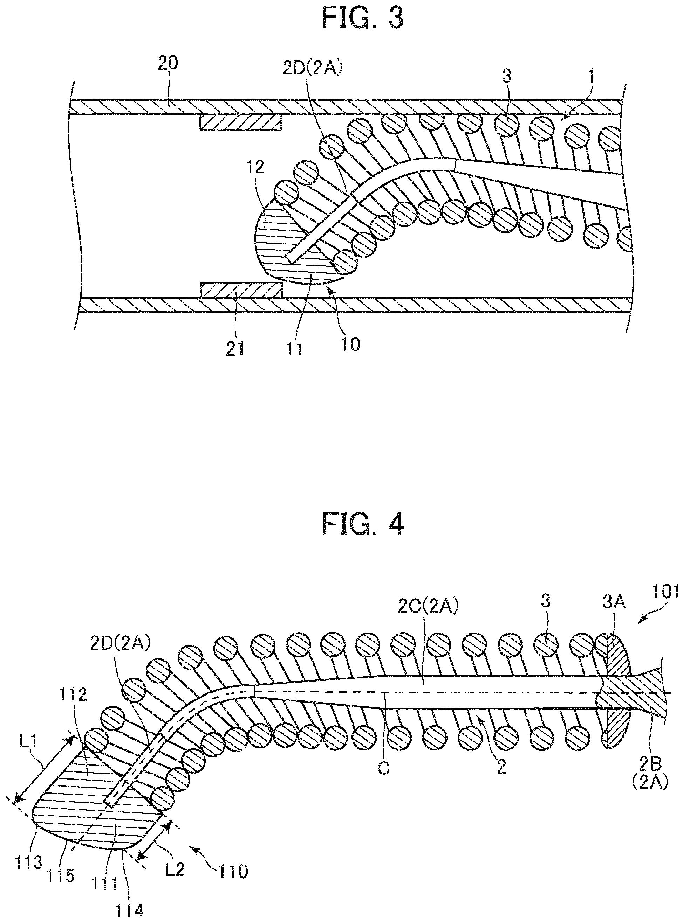

According to this configuration, the outer shape of the curve inner portion 11 on the distal end side has a smaller projection height to the outside than the outer shape of the curve outer portion 12 on the distal end side. Thus, when the guide wire 1 is inserted into a device (an inserted member) having a convex portion on the inner peripheral surface, the curve inner portion 11 can prevent the distal tip 10 from being caught by the convex portion and thus can prevent that the advancement of the guide wire 1 is obstructed. That is, as shown in FIG. 3, when the guide wire 1 is inserted into a catheter 20 having a marker 21 on its inner peripheral surface, the curve inner portion 11 can prevent the distal tip 10 from being caught by the marker 21.

The outer shape of the curve inner portion 11 on the distal end side includes the first arcuate portion 11A having the first curvature R1, and the outer shape of the curve outer portion 12 on the distal end side includes the second arcuate portion 12A having the second curvature R2. The first curvature R1 is configured to be smaller than the second curvature R2. This makes the outer shape of the curve inner portion 11 on the distal end side gentler than the outer shape of the curve outer portion 12 on the distal end side, allowing the curve inner portion 11 to prevent the distal tip 10 from being caught by the marker 21.

### <Second embodiment>

Next, a guide wire according to a second embodiment of the present invention will be described with reference to the drawings. The same members as those of the guide wire 1 in the first embodiment are denoted by the same reference numerals, and the description thereof is omitted, and the configuration different from that of the guide wire 1 in the first embodiment will be described.

FIG. 4 is a partial cross-sectional view of a guide wire 101 according to a second embodiment. As shown in FIG. 4, the shape of a distal tip 110 is different from the shape of the distal tip 10 in the first embodiment.

The distal tip 110 has a shape formed by obliquely cutting a cylinder. In the longitudinal section of the core shaft 2 (cross section cut along the plane including the central axis C of the core shaft 2), the distal tip 110 includes a curve inner portion 111 located on the inside of the curve of the core shaft 2 relative to the distal end (the center of the distal end) of the core shaft 2 and a curve outer portion 112 located on the outside of the curve of the core shaft 2 relative to the distal end (the center of the distal end) of the core shaft 2. In the distal tip 110 shown in FIG. 4, a part located on the inside of the curve of the core shaft 2 relative to the central axis C corresponds to the curve inner portion 111, and a part located on the outside of the curve of the core shaft 2 corresponds to the curve outer portion 112. The cross-sectional area of the curve inner portion 111 is configured to be smaller than the cross-sectional area of the curve outer portion 112.

The length from the distal end of the coil body 3 to the distal end surface of the distal tip 110 along the central axis C is defined as a projection length. The distal tip 110 includes a distal portion 113 provided in the curve outer portion 112 and having the longest projection length L1, and a proximal portion 114 provided in the curve inner portion 111 and having a projection length L2 shorter than the projection length L1 of the distal portion 113. In the distal tip 110, the projection length is configured to decrease from the distal portion 113 toward the proximal portion 114.

The distal tip 110 includes a flat portion 115 on the distal end side. The flat portion 115 is inclined relative to the axis (central axis C) of the distal end portion of the core shaft 2 so that a part on the curve inner portion 111 side is closer to the coil body 3 than a part on curve outer portion 112 side.

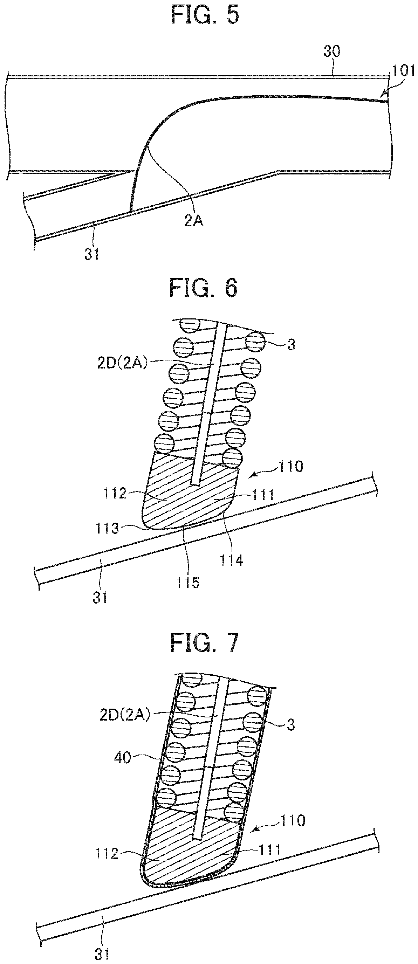

FIGS. 5 and 6 are explanatory diagrams of how the guide wire 101 is used. As shown in FIG. 5, when using the guide wire 101, if a side branch 31 is selected from a main vessel 30 with a large blood vessel diameter, the distal end portion 2A is bent at a sharp angle, and the distal tip 110 is brought into contact with the inner surface of the side branch 31 at a nearly perpendicular angle. The distal tip 110 is configured such that the projection length becomes shorter from the distal portion 113 toward the proximal portion 114 and includes the flat portion 115 on the distal end side. Thus, the distal tip 110 is brought into contact with the inner surface of the side branch 31 in a state close to a plane contact. Thus, the distal tip 110 can be prevented from damaging the inner surface of the side branch 31.

The guide wire 101 of the present embodiment also has the same effects as the guide wire 1 of the first embodiment. Further, similarly to the guide wire 101 of the present embodiment, the guide wire 1 of the first embodiment can prevent the distal tip 10 from damaging the inner surface of the side branch 31 when it is used in the blood vessel and the side branch 31 is selected from the main vessel 30.

Note that the present invention is not limited to the configurations of the above-mentioned embodiments, but is defined by the scope of the claims, and is intended to include all modifications within the meaning and scope of equivalents to the scope of claims.

Although the tubular body is the coil body 3 in the above-mentioned embodiments, it may be a braided braid, a resin tube, or the like. The flat portion 115 does not need to be flat and may be gently convex. As shown in FIG. 7, the surfaces of the distal tip 110 and the coil body 3 may be coated with a hydrophilic resin film 40. Due to the lubricating property of the resin film 40, the guide wire 101 can be advanced with little resistance even if the force vector in the advancing direction of the guide wire 101 is small. In this case, a part of the distal tip 110 covered with the resin film 40 can be brought into plane contact with the blood vessel inner wall. This can reduce the pressure on the resin film 40 due to the contact with the blood vessel wall compared to a case where the part covered with the resin film 40 is brought into contact with the inner surface of the side branch 31 in a state far from a plane contact, for example, in a state close to a point contact. Thus, it is possible to prevent the crush of the resin film 40 or the release of moisture contained in the resin film 40, thereby reducing deterioration of the lubricating property of the guide wire 101. Note that the surfaces of the distal tip 10 and the coil body 3 in the first embodiment may be coated with a hydrophilic resin film.

### DESCRIPTION OF REFERENCE NUMERALS

1, 101: Guide wire
2: Core shaft
2A: Distal end portion
2D: Most distal end portion
3: Coil body
10, 110: Distal tip
11, 111: Curve inner portion
12, 112: Curve outer portion
11A: First arcuate portion
12A: Second arcuate portion
113: Distal portion
114: Proximal portion

## Claims

1. A guide wire comprising:
a core shaft having a distal end portion which is curved in a predetermined direction;
a tubular body covering an outer periphery of the core shaft; and
a distal tip joining the distal end portion of the core shaft and a distal end portion of the tubular body,
wherein the distal tip has a convex shape toward a distal end side, and includes, in a longitudinal section of the core shaft, a curve inner portion located on an inside of the curve of the core shaft relative to a distal end of the core shaft and a curve outer portion located on an outside of the curve of the core shaft relative to the distal end of the core shaft, and
a cross-sectional area of the curve inner portion is smaller than a cross-sectional area of the curve outer portion.

2. The guide wire according to claim 1, wherein:
an outer shape of the curve inner portion on a distal end side includes a first arcuate portion having a first curvature; and
an outer shape of the curve outer portion on a distal end side includes a second arcuate portion having a second curvature, the first curvature being smaller than the second curvature.

3. The guide wire according to claim 1, wherein, provided that a length from a distal end of the tubular body to a distal end surface of the distal tip is defined as a projection length, the distal tip includes:
a distal portion provided in the curve outer portion and having a longest projection length; and
a proximal portion provided in the curve inner portion and having a shorter projection length than that of the distal portion, the projection length becoming shorter from the distal portion toward the proximal portion.

4. The guide wire according to claim 3, wherein:
the distal tip includes a flat portion, and
the flat portion is inclined relative to an axis of the distal end portion of the core shaft so that a part of the flat portion on a side of the curve inner portion is closer to the tubular body than a part of the flat portion on a side of the curve outer portion.
